# EUROPEAN PATENT APPLICATION

(11) **EP 2 537 828 A1**
(43) Date of publication of application: **26.12.2012**
(21) Application number: 11742383.0
(22) Date of filing: 10.02.2011
(51) Int. Cl.: C07C 253/34, C07C 255/31

(54) **METHOD FOR PRODUCING CYANOALKENYLCYCLOPROPANECARBOXYLIC ACID SALT**

(30) Priority: 12.02.2010 JP 2010028640
(71) Applicant: Sumitomo Chemical Company, Limited, Tokyo 104-8260 (JP)
(72) Inventor: KOMOTO, Ichiro, Nishinomiya-shi Hyogo 662-0083 (JP); SOMYO, Toshio, Ibaraki-shi Osaka 567-0841 (JP); HIROTA, Masaji, Ibaraki-shi Osaka 567-0841 (JP)
(74) Representative: Vossius & Partner
(86) International application number: PCT/JP2011/053431
(87) International publication number: WO 2011/099645

(57) **Abstract**

A method for producing a salt of an amine with a cyanoalkenylcyclopropanecarboxylic acid, the method comprising a first step of mixing a carboxylic acid compound represented by formula (2C) and at least one amine selected from the group consisting of primary amines and secondary amines in an organic solvent; a second step of depositing a salt of the amine with a cyanoalkenylcyclopropanecarboxylic acid represented by formula (2) from the mixture obtained in the first step; and a third step of recovering the salt deposited in the second step; 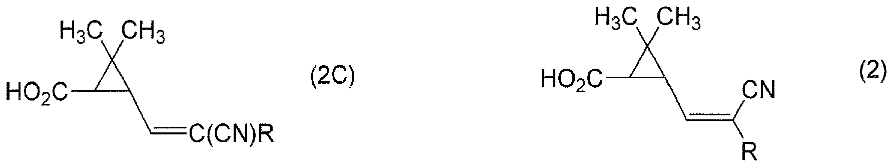 wherein, R represents an alkyl group optionally having a substituent or a halogen atom.

## Description

### Technical Field

The present invention relates to a method for producing a salt of an amine with a cyanoalkenylcyclopropanecarboxylic acid.

### Background Art

A cyanoalkenylcyclopropanecarboxylic acid represented by formula (2): 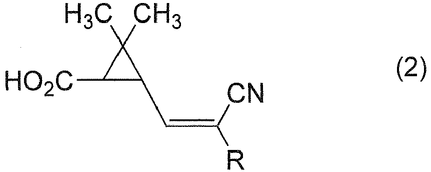 wherein, R represents an alkyl group optionally having a substituent or a halogen atom (hereinafter, referred to as carboxylic acid (2) in some cases), such as (Z)-2,2-dimethyl-3-[2-cyano-1-propen-1-yl]cyclopropanecarbo xylic acid or the like is useful as a synthetic intermediate for agents of controlling pests such as harmful insects and the like (see, e.g., JP 2008-239597 A) .

As the method for producing such a carboxylic acid (2) , Agr. Biol. Chem. , 34, 1119 (1970) describes a method of hydrolyzing (Z)-2,2-dimethyl-3-[2-cyano-1-propen-1-yl]cyclopropanecarbo xylic acid tert-butyl ester to obtain (Z)-2,2-dimethyl-3-[2-cyano-1-propen-1-yl]cyclopropanecarbo xylic acid as the carboxylic acid (2).

### Summary of the Invention

The present invention is as described below.
[1] A method for producing a salt of an amine with a cyanoalkenylcyclopropanecarboxylic acid, the method comprising
   a first step of mixing a carboxylic acid compound represented by formula (2C) : 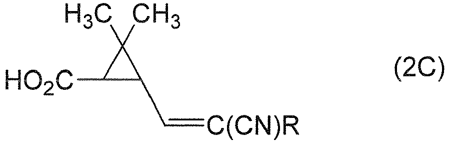 wherein, R represents an alkyl group optionally having a substituent or a halogen atom,
   and at least one amine selected from the group consisting of primary amines and secondary amines in an organic solvent;
   a second step of depositing a salt of the amine with a cyanoalkenylcyclopropanecarboxylic acid represented by formula (2) : 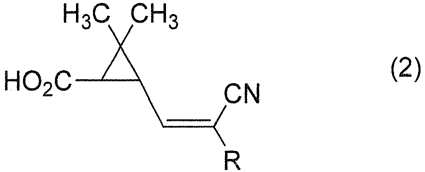 wherein, R represents the same meaning as described above, from the mixture obtained in the first step; and
   a third step of recovering the salt deposited in the second step.
[2] The method according to [1] , wherein the carboxylic acid compound represented by formula (2C) is a mixture of a cyanoalkenylcyclopropanecarboxylic acid represented by formula (2) and a cyanoalkenylcyclopropanecarboxylic acid represented by formula (1) : 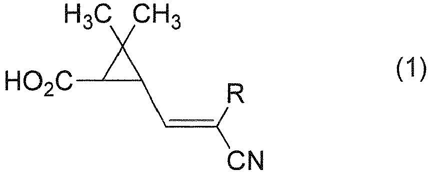 wherein, R represents the same meaning as described above.
[3] The method according to [1] or [2], wherein the amine is selected from the group consisting of t-butylamine, n-butylamine, hexylamine, cyclohexylamine and morpholine.
[4] The method according to any one of [1] to [3], wherein the organic solvent is a mixed solvent composed of a hydrocarbon solvent and an alcohol solvent having 1 to 4 carbon atoms or a hydrocarbon solvent.
[5] The method according to any one of [2] to [4], wherein the carboxylic acid compound represented by formula (2C) is one obtained via a step of isomerizing a cyanoalkenylcyclopropanecarboxylic acid represented by formula (1) into a cyanoalkenylcyclopropanecarboxylic acid represented by formula (2).
[6] A salt of at least one amine selected from the group consisting of t-butylamine, n-butylamine, hexylamine, cyclohexylamine and morpholine with a cyanoalkenylcyclopropanecarboxylic acid represented by formula (2) : 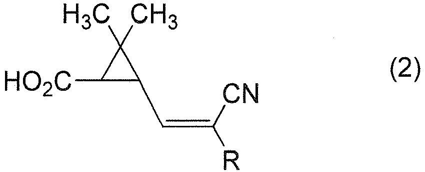 wherein, R represents an alkyl group optionally having a substituent or a halogen atom.
[7] A method for producing a cyanoalkenylcyclopropanecarboxylic acid, the method comprising a step of mixing a salt of an amine with a cyanoalkenylcyclopropanecarboxylic acid represented by formula (2) : 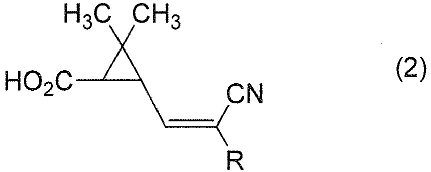 wherein, R represents an alkyl group optionally having a substituent or a halogen atom,
   the salt having been obtained by the method according to any one of [1] to [5], and an aqueous alkali solution and/or a water-soluble acid.

### Modes for Carrying Out the Invention

The present invention will be illustrated in detail below.

The first step of the present invention is a step of mixing a carboxylic acid compound represented by formula (2C): 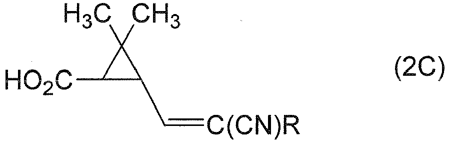 (hereinafter, referred to as carboxylic acid (2C) in some cases) and at least one amine selected from the group consisting of primary amines and secondary amines in an organic solvent.

In formula (2C), R represents an alkyl group optionally having a substituent or a halogen atom.

The halogen atom includes a fluorine atom, a chlorine atom, a bromine atom, an iodine atom and the like, preferably a chlorine atom and a bromine atom.

The alkyl group means a linear alkyl group having about 1 to about 10 carbon atoms, a branched alkyl group having about 3 to about 10 carbon atoms or a cycloalkyl group having about 3 to about 10 carbon atoms. Examples of the linear alkyl group include a methyl group, an ethyl group and a n-propyl group. Examples of the branched alkyl group include an isopropyl group, an isobutyl group, a t-butyl group, an amyl group and a 2-ethylhexyl group. Examples of the cycloalkyl group include a cyclopropyl group, a cyclopentyl group and a cyclohexyl group. The substituent optionally carried on the alkyl group includes halogen atoms, alkoxy groups having about 1 to about 4 carbon atoms and the like, and the alkyl group having the substituent includes a fluoromethyl group, a chloromethyl group, a trifluoromethyl group, a trichloromethyl group and the like.

R includes preferably linear alkyl groups having 1 to 4 carbon atoms, a chlorine atom, a bromine atom, and the like, more preferably a methyl group and a chlorine atom, and the like.

The carboxylic acid (2C) contains a cyanoalkenylcyclopropanecarboxylic acid represented by formula (2): 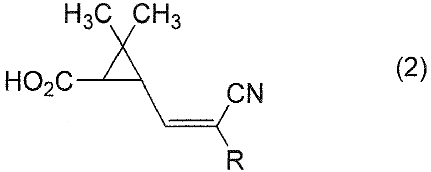 wherein, R represents the same meaning as described above, (hereinafter, referred to as carboxylic acid (2) in some cases) , and further, may contain a cyanoalkenylcyclopropanecarboxylic acid represented by formula (1) : 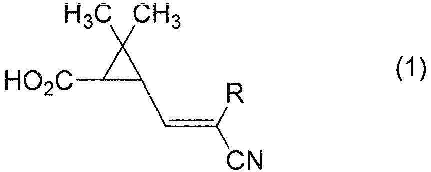 wherein, R represents the same meaning as described above, (hereinafter, referred to as carboxylic acid (1) in some cases) .

The content of the carboxylic acid (2) in the carboxylic acid (2C) is preferably 40 to 100 wt%, and the content of the carboxylic acid (1) in the carboxylic acid (2C) is preferably 0 to 60 wt%.

Here, the carboxylic acid (1) is the compound in which the cyano group and the cyclopropane ring are present on the opposite sides (trans) across the double bond.

Of the carbon atoms forming the cyclopropane ring of the carboxylic acid (1) , both the carbon atom linked to the carboxyl group (-CO₂H) and the carbon atom linked to the alkenyl group (-C=C(CN)R) are the asymmertic carbon atoms, and the carboxylic acid (1) having the asymmertic carbon atoms may be either optically active or optically inactive.

The carboxylic acid (1) includes isomers represented by formula (1') : 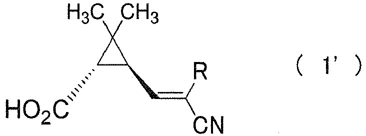 wherein, R represents the same meaning as described above, and enantiomers thereof, namely, isomers in which the alkenyl group (-C=C(CN)R) and the carboxyl group (-CO₂H) are present on the opposite sides (trans) across the cyclopropane ring plane (hereinafter, referred to collectively as cyclopropane trans form (1' ) in some cases), and isomers represented by formula (1'' ) : 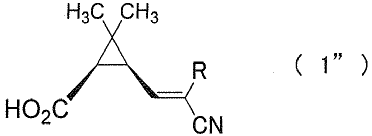 wherein, R represents the same meaning as described above, and enantiomers thereof, namely, isomers in which the alkenyl group (-C=C(CN)R) and the carboxyl group (-CO₂H) are present on the same side (cis) of the cyclopropane ring plane (hereinafter, referred to collectively as cyclopropane cis form (1'' ) in some cases).

As the carboxylic acid (1) , those rich in the cyclopropane trans form (1' ) are preferable, and more preferably, the content of the cyclopropane trans form (1' ) is 90 wt% or more with respect to the total amount of the carboxylic acid (1).

The carboxylic acid (1) includes compounds represented by the following formulae (1-1) to (1-16), namely, (E)-2,2-dimethyl-3-[2-cyano-1-propen-1-yl]cyclopropanecarbo xylic acid (1-1), (E)-2,2-dimethyl-3-[2-cyano-1-buten-1-yl]cyclopropanecarbox ylic acid (1-2), (E)-2,2-dimethyl-3-[2-cyano-1-penten-1-yl]cyclopropanecarbo xylic acid (1-3), (E)-2,2-dimethyl-3-[2-cyano-1-hexen-1-yl]cyclopropanecarbox ylic acid (1-4), (E)-2,2-dimethyl-3-[2-cyano-4-methyl-1-penten-1-yl]cyclopro panecarboxylic acid (1-5), (E)-2,2-dimethyl-3-[2-cyano-3-methyl-1-buten-1-yl]cycloprop anecarboxylic acid (1-6), (E)-2,2-dimethyl-3-[2-cyano-3,3-dimethy-1-1-buten-1-yl]cyclo propanecarboxylic acid (1-7), (E)-2,2-dimethyl-3-[2-cyano-1-hepten-1-yl]cyclopropanecarbo xylic acid (1-8), (E)-2,2-dimethyl-3-[2-cyano-4-ethyl-1-octen-1-yl]cyclopropa necarboxylic acid (1-9), (E)-2,2-dimethyl-3-[2-cyano-2-cyclopropylvinyl]cyclopropane carboxylic acid (1-10), (E)-2,2-dimethyl-3-[2-cyano-2-cyclopentylvinyl]cyclopropane carboxylic acid (1-11), (E)-2,2-dimethyl-3-[2-cyano-2-cyclohexylvinyl]cyclopropanec arboxylic acid (1-12), (Z)-2,2-dimethyl-3-[2-cyano-2-fluorovinyl]cyclopropanecarbo xylic acid (1-13), (2)-2,2-dimethyl-3-[2-chloro-2-cyanovinyl]cyclopropanecarbo xylic acid (1-14), (Z)-2,2-dimethyl-3-[2-bromo-2-cyanovinyl]cyclopropanecarbox ylic acid (1-15) and (Z)-2,2-dimethyl-3-[2-cyano-2-iodovinyl]cyclopropanecarboxy lic acid (1-16).

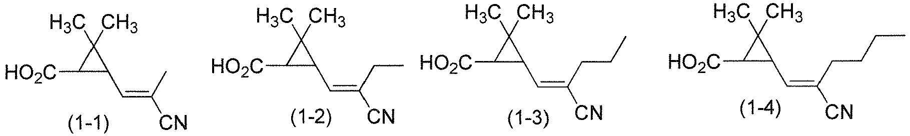

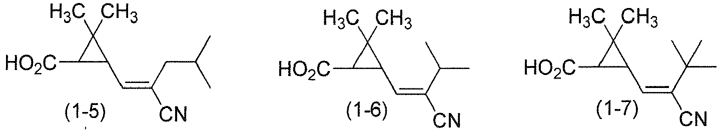

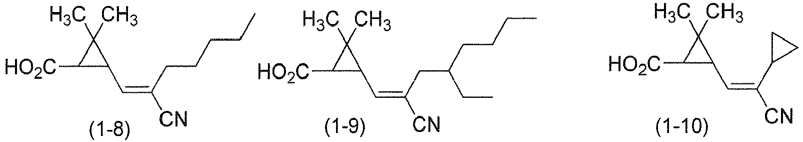

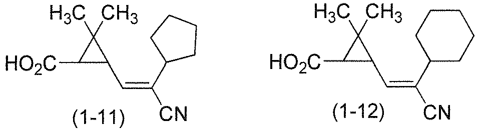

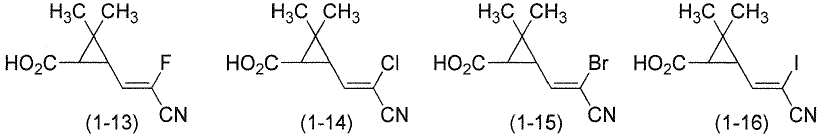

The carboxylic acid (1) includes preferably (E)-2,2-dimethyl-3-[2-cyano-l-propen-1-yl]cyclopropanecarbo xylic acid (1-1), (E)-2,2-dimethyl-3-[2-cyano-l-buten-l-yl]cyclopropanecarbox ylic acid (1-2), (E)-2,2-dimethyl-3-[2-cyano-1-penten-1-yl]cyclopropanecarbo xylic acid (1-3), (E)-2,2-dimethyl-3-[2-cyano-1-hexen-1-yl]cyclopropanecarbox ylic acid (1-5), (E)-2,2-dimethyl-3-[2-cyano-3-methyl-1-buten-1-yl]cycloprop anecarboxylic acid (1-6), (E)-2,2-dimethyl-3-[2-cyano-3,3-dimethyl-1-buten-1-yl]cyclo propanecarboxylic acid (1-7), (Z)-2,2-dimethyl-3-[2-chloro-2-cyanovinyl]cyclopropanecarbo xylic acid (1-14), (Z)-2,2-dimethyl-3-[2-bromo-2-cyanovinyl]cyclopropanecarbox ylic acid (1-15) and the like, more preferably (E)-2,2-dimethyl-3-[2-cyano-1-propen-1-yl]cyclopropanecarbo xylic acid (1-1), (Z)-2,2-dimethyl-3-[2-chloro-2-cyanovinyl]cyclopropanecarbo xylic acid (1-14) and the like.

The carboxylic acid (2) is a compound in which the cyano group and the cyclopropane ring are present on the same side (cis) of the double bond.

Of the carbon atoms forming the cyclopropane ring of the carboxylic acid (2) , both the carbon atom linked to the carboxyl group (-CO₂H) and the carbon atom linked to the alkenyl group are the asymmertic carbon atoms, and the carboxylic acid (2) having the asymmertic carbon atoms may be either optically active or optically inactive.

The carboxylic acid (2) includes isomers represented by formula (2' ) : 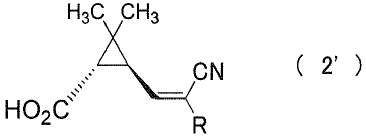 wherein, R represents the same meaning as described above, and enantiomers thereof, namely, isomers in which the alkenyl group (-C=C(CN)R) and the carboxyl group are present on the opposite sides (trans) across the cyclopropane ring plane (hereinafter, referred to collectively as cyclopropane trans form (2') in some cases) , and isomers represented by formula (2'' ) : 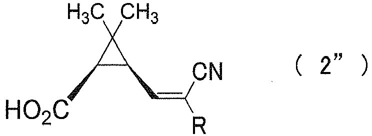 wherein, R represents the same meaning as described above, and enantiomers thereof, namely, isomers in which the alkenyl group (-C=C(R)-CN) and the carboxyl group are present on the same side (cis) of the cyclopropane ring plane (hereinafter, referred to collectively as cyclopropane cis form (2 '' ) in some cases).

As the carboxylic acid (2), those rich in the cyclopropane trans form (2' ) are preferable, and more preferably, the content of the cyclopropane trans form (2' ) is 90 wt% or more with respect to the total amount of the carboxylic acid (2).

The carboxylic acid (2) includes compounds represented by the following formulae (2-1) to (2-16), namely, (Z)-2,2-dimethyl-3-[2-cyano-1-propen-1-yl]cyclopropanecarbo xylic acid (2-1), (Z)-2,2-dimethyl-3-[2-cyano-1-buten-1-yl]cyclopropanecarbox ylic acid (2-2), (Z)-2,2-dimethyl-3-[2-cyano-1-penten-1-yl]cyclopropanecarbo xylic acid (2-3) , (Z)-2,2-dimethyl-3-[2-cyano-1-hexen-1-yl]cyclopropanecarbox ylic acid (2-4), (Z)-2,2-dimethyl-3-[2-cyano-4-methyl-1-penten-1-yl]cyclopro panecarboxylic acid (2-5), (Z)-2,2-dimethyl-3-[2-cyano-3-methyl-1-buten-1-yl]cycloprop anecarboxylic acid (2-6), (Z)-2,2-dimethyl-3-[2-cyano-3,3-dimethyl-1-buten-1-yl]cyclo propanecarboxylic acid (2-7), (Z)-2,2-dimethyl-3-[2-cyano-1-hepten-1-yl]cyclopropanecarbo xylic acid (2-8), (Z)-2,2-dimethyl-3-[2-cyano-4-ethyl-1-octen-1-yl]cyclopropa necarboxylic acid (2-9), (Z)-2,2-dimethyl-3-[2-cyano-2-cyclopropylvinyl]cyclbpropane carboxylic acid (2-10), (2)-2,2-dimethyl-3-[2-cyano-2-cyclopentylvinyl]cyclopropane carboxylic acid (2-11), (Z)-2,2-dimethyl-3-[2-cyano-2-cyclohexylvinyl]cyclopropanec arboxylic acid (2-12), (E)-2,2-dimethyl-3-[2-cyano-2-fluorovinyl]cyclopropanecarbo xylic acid (2-13), (E)-2,2-dimethyl-3-[2-chloro-2-cyanovinyl]cyclopropanecarbo xylic acid (2-14), (E)-2,2-dimethyl-3-[2-bromo-2-cyanovinyl]cyclopropanecarbox ylic acid (2-15), (E)-2,2-dimethyl-3-[2-cyano-2-iodovinyl]cyclopropanecarboxy lic acid (2-16) and the like.

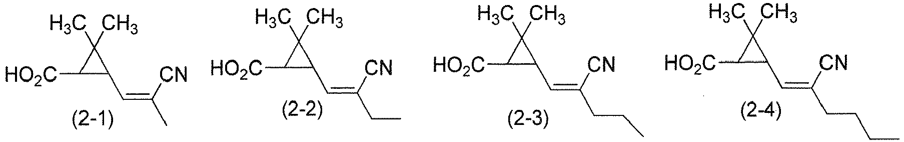

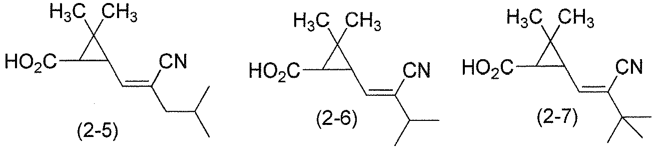

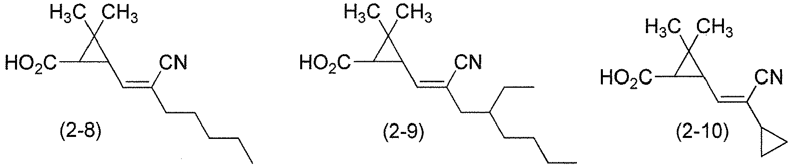

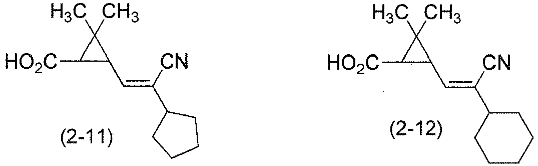

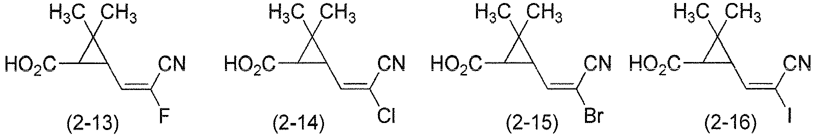

The carboxylic acid (2C) used in the present invention is exemplified those containing a carboxylic acid (2) obtained by the following preparation method, and the like.

When the carboxylic acid (2C) contains the carboxylic acid (1) and the carboxylic acid (2), it is preferable that the carboxylic acid (1) and the carboxylic acid (2) have the same structure excepting a difference in whether the cyano group and the cyclopropane ring are present on the same side of or opposite sides across the double bond. Specifically, the carboxylic acid (2C) preferably contains a compound represented by formula (1-1) and a compound represented by formula (2-1). Further, specifically, the carboxylic acid (2C) preferably contains a compound represented by formula (1-2) and a compound represented by formula (2-2).

The method for preparingof the carboxylic acid (2C) includes methods described in non-patent document 1, that is,
a method that involves reacting cyclopropanealdehyde represented by formula (3) : 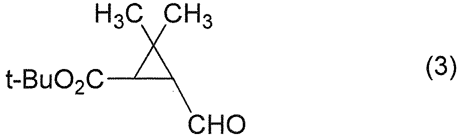 and a phosphonate compound represented by formula (4) : 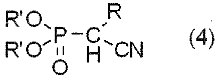 wherein, R represents the same meaning as described above and R' represents an alkyl group having 1 to 4 carbon atoms such as a methyl group, an ethyl group and the like,
to obtain a cyanoalkenylcyclopropanecarboxylic acid ester represented by formula (5) : 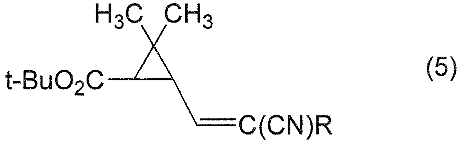 , subsequently, converting the ester into a carboxylic acid by using an acid such as p-toluenesulfonic acid and the like, thereby producing a carboxylic acid (2C) ;
a method in which cyclopropanealdehyde represented by formula (6) : 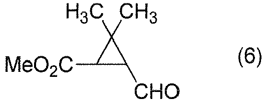 and an aldehyde compound represented by formula (7): 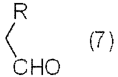 wherein, R represents the same meaning as described above, are condensation-reacted in the presence of a base, to obtain an unsaturated aldehyde compound represented by formula (8) : 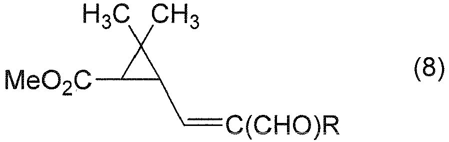 wherein, R represents the same meaning as described above, the unsaturated aldehyde compound is further condensed with hydroxylamine or a hydrochloride or hydrosulfate thereof, or the like, to obtain an oxime compound represented by formula (9) : 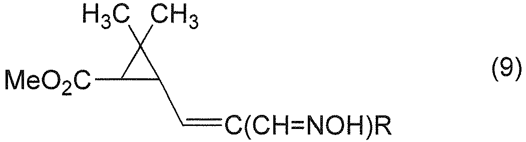 wherein, R represents the same meaning as described above, further it is reacted with a dehydrating agent in the presence of a base, to obtain a cyanoalkenylcyclopropanecarboxylic acid ester represented by formula (10): 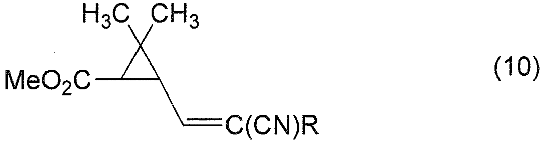 , further, hydrolysis of the ester is performed with an aqueous solution of an alkali metal hydroxide such as sodium hydroxide, potassium hydroxide and the like, to produce a carboxylic acid (2C) ;
a method in which cyclopropanealdehyde represented by formula (6) and a compound represented by formula (11) : 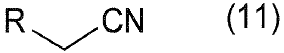 wherein, R represents the same meaning as described above, are reacted in the presence of a base, to obtain a cyanoalkenylcyclopropanecarboxylic acid ester represented by formula (10) , further, hydrolysis of the ester is performed with an aqueous solution of an alkali metal hydroxide such as sodium hydroxide, potassium hydroxide and the like, to produce a carboxylic acid (2C) ;
a method in which cyclopropanealdehyde represented by formula (6) and 2-cyanopropionic acid are reacted in the presence of a base, to obtain a cyanoalkenylcyclopropanecarboxylic acid ester represented by formula (10) , further, hydrolysis of the ester is performed with an aqueous solution of an alkali metal hydroxide such as, for example, sodium hydroxide, potassium hydroxide and the like, to produce a carboxylic acid (2C);
and other methods.

As the carboxylic acid (2C), also those obtained via a step of isomerizing a carboxylic acid (1) into a carboxylic acid (2) (hereinafter, referred to as isomerization step in some cases) are preferably used. The isomerization step includes, for example, the following step.

Isomerization step: a step of isomerizing a carboxylic acid (1) to obtain a carboxylic acid (2) in the presence of at least one isomerization catalyst selected from the group consisting of bromine, hydrogen bromide, carboxylic bromides, phosphorus bromide compounds, N-brominated imide compounds, N-brominated amide compounds, brominated alkylsilane compounds, thionyl bromide, boron bromide compounds, aluminum bromide compounds, thiol compounds, disulfide compounds, thiocarboxylic acid compounds, nitric acid and nitirate salts.

The isomerization catalyst in the isomerization step includes preferably, for example, bromine, hydrogen bromide, phosphorus tribromide, diphenyl sulfide and the like.

The used amount of the isomerization catalyst is in the range of usually 0.01 to 0.5 mol, preferably 0.05 to 0.2 mol with respect to 1 mol of the sum of a carboxylic acid (1) and a carboxylic acid (2).

When hydrogen bromide is used as the isomerization catalyst, it is preferably used in the form of a hydrogen bromide-containing acetic acid solution or a hydrobromic acid aqueous solution. When a hydrobromic acid aqueous solution is used as the isomerization catalyst and an organic solvent immiscible with water (for example, aromatic hydrocarbons, halogenated aromatic hydrocarbons, aliphatic hydrocarbons, alicyclic hydrocarbons and the like) is used together as the solvent, the above-described reaction can also be progressed smoothly by allowing an inorganic salt showing large solubility in water and not disturbing the reaction to exist in the reaction system. Specific examples thereof include lithium bromide, lithium chloride, calcium bromide, calcium chloride, magnesium bromide, magnesium chloride and magnesium sulfate.

In the isomerization step, an isomerization catalyst may only be used, and it is preferable that an isomerization catalyst and a radical initiator are used together. Here, the radical initiator means a compound which is difference from the isomerization catalyst and capable of generating a radical.

The radical initiator includes inorganic peroxides such as ammonium persulfate, potassium persulfate, sodium persulfate, hydrogen peroxide and the like; ketone peroxide compounds such as methyl ethyl ketone peroxide, cyclohexanone peroxide and the like; hydroperoxide compounds such as t-butyl hydroperoxide, 1,1,3,3-tetramethylbutyl hydroperoxide, cumene hydroperoxide, triphenylmethyl peroxide and the like; diacyl peroxide compounds such as benzoyl peroxide (dibenzoyl peroxide), diacetyl peroxide and the like; peracid ester compounds such as t-butyl perbenzoate, t-butyl peracetate and the like; dialkyl peroxides such as di-t-butyl peroxide and the like; peracid compounds such as peracetic acid, perbenzoic acid, m-chlorobenzoic acid and the like; azo compounds such as azobisisobutyronitrile, 2,2'-azobis(4-methoxy-2,4-dimethylvaleronitrile), 2,2'-azobis(2,4-dimethylvaleronitrile), 1,1'-azobis(cyclohexane-1-carbonitrile), 4,4'-azobis(4-cyanopentanoic acid), 1-[(1-cyano-1-methylethyl)azo]formamide, 2,2'-azobis(2-amidinopropane) , 2,2'-azobis[2-methyl-N-(2-hydroxyethyl)propionamide] , methyl azobisisobutyrate and the like; etc.

The radical initiator includes preferably t-butyl hydroperoxide, benzoyl peroxide, azobisisobutyronitrile, 2,2'-azobis(4-methoxy-2,4-dimethylvaleronitrile) and the like.

The used amount of the radical initiator in the case of use of an isomerization catalyst is usually 0 to 0.3 mol, preferably 0.01 to 0.1 mol with respect to 1 mol of the sum of a carboxylic acid (1) and a carboxylic acid (2).

The reaction temperature in the isomerization step is in the range of usually 0 to 80°C, preferably 20 to 60°C.

The reaction time in the isomerization step is in the range of usually 1 minute to 24 hours, preferably 1 hour to 10 hours.

Though the isomerization step can be carried out in the presence of no solvent at temperatures not lower than the melting points of a carboxylic acid (1) and a carboxylic acid (2), it is preferable that the isomerization step is carried out in the presence of a solvent irrespective of the reaction temperature of the isomerization step.

The solvent used in the isomerization step includes aromatic hydrocarbons such as toluene, xylene, ethylbenzene and the like; aliphatic hydrocarbons such as n-pentane, n-hexane and the like; alicyclic hydrocarbons such as cyclopentane, cyclohexane and the like; halogenated aromatic hydrocarbons such as monochlorobenzene and the like; ether compounds such as diethyl ether, tert-butyl methyl ether, 1,2-dimethoxyethane, 1,4-dioxane, tetrahydrofuran, anisole and the like.

The used amount of the solvent is in the range of usually 0.50 to 20 parts by weight with respect to 1 part by weight of the sum of a carboxylic acid (1) and a carboxylic acid (2).

Further in detail, the isomerization step includes a method in which an isomerization catalyst and a radical initiator are gradually mixed in a mixed solution of a carboxylic acid (1), a carboxylic acid (2) and an organic solvent, then, the mixture is stirred to obtain a carboxylic acid (2C) having high content of a carboxylic acid (2), and the like.

The content of a carboxylic acid (2) contained in a carboxylic acid (2C) obtained via the isomerization step varies depending on the kinds of a carboxylic acid (1) and a carboxylic acid (2), and is higher than the content before the isomerization step and usually 1 to 80 wt%, preferably 30 to 80 wt%, more preferably 50 to 70 wt%. Regarding the steric configuration of an asymmertic carbon atom contained in a cyclopropane ring of a carboxylic acid (2) obtained from a carboxylic acid (1) in the isomerization step, the same steric configuration as that of the carboxylic acid (1) used in the isomerization step is approximately kept.

For example, regarding (E)-2,2-dimethyl-3-[2-cyano-l-propen-1-yl]cyclopropanecarbo xylic acid (1-1), a carboxylic acid in which 1-position and 3-position of a cyclopropane ring are both in R configuration (1R, 3R) among the carboxylic acids (1-1) gives, via the isomerization step, (Z)-2,2-dimethyl-3-[2-cyano-1-propen-1-yl]cyclopropanecarbo xylic acid (2-1) in which 1-position and 3-position of a cyclopropane ring are both kept approximately in R configuration (1R, 3R).

Regarding the structural configuration of an alkenyl group (-C=C (CN) R) and a carboxyl group with respect to a cyclopropane ring in a carboxylic acid (2) obtained from a carboxylic acid (1) in the isomerization step, the same structural configuration as that of the carboxylic acid (1) used in the isomerization step is approximately kept. That is, the proportion of the sum of a cyclopropane trans form (1' ) and a cyclopropane trans form (2') to the sum of a carboxylic acid (1) and a carboxylic acid (2) before the isomerization step, and the proportion of the sum of a cyclopropane trans form (1' ) and a cyclopropane trans form (2') to the sum of a carboxylic acid (1) and a carboxylic acid (2) in a carboxylic acid (2C) obtained via the isomerization step are approximately identical.

A mixture after completion of the isomerization step may be subjected to a post treatment such as neutralization, extraction, water-washing and the like, and of course, a mixture after the post treatment may be subjected to a purification treatment such as crystallization purification, extraction purification, distillation purification, adsorption purification on activated carbon, silica, alumina and the like, chromatographic methods such as silica gel column chromatography and the like.

In at least one amine selected from the group consisting of primary amines and secondary amines used in the first step (hereinafter, referred to as amine compound in some cases), the primary amine includes compounds obtained by substituting one hydrogen atom of an ammonia molecule by a linear alkyl group having 3 to 12 carbon atoms, a branched alkyl group or an alicyclic alkyl group, such as propylamine, n-butylamine, sec-butylamine, t-butylamine, 2-ethylhexylamine, cyclohexylamine and the like; etc.

A hydrogen atom contained in the alkyl group may be substituted by an aromatic group, and the alkyl group substituted by an aromatic group includes benzylamine and the like. Further, -CH₂- contained in the alkyl group may be substituted by an oxygen atom (-0-).

Of the amine compounds, the secondary amine includes, for example, compounds obtained by substituting two hydrogen atoms of an ammonia molecule by a linear alkyl group having 4 to 12 carbon atoms or a branched alkyl group, compounds obtained by substituting two hydrogen atoms of an ammonia molecule by a linear alkylene group having 4 to 12 carbon atoms or a branched alkylene group, such as diethylamine, diisopropylamine, diisobutylamine, di-2-ethylhexylamine, pyrrolidine, piperidine, pipecoline and the like; etc.

A hydrogen atom contained in the alkyl group or the alkylene group may be substituted by an aromatic group, and -CH₂- contained in the alkyl group or the alkylene group may be substituted by an oxygen atom (-0-) The secondary amine includes morpholine obtained by substituting -CH₂- contained in pyrrolidine by an oxygen atom (-0-), and the like.

The amine compound includes preferably t-butylamine, n-butylamine, hexylamine, cyclohexylamine or morpholine.

The used amount of the amine compound varies depending on the kind and the amount of an organic solvent described later, and is usually 0.4 to 1.2 mol, preferably 0.5 to 1.0 mol with respect to 1 mol of a carboxylic acid (2) contained in a carboxylic acid (2C).

The organic solvent used in the first step is, for example, an organic solvent capable of dissolving an amine compound and a carboxylic acid (2C), and includes organic solvents exemplified in the above-described isomerization step. Such organic solvents include, preferably, aromatic hydrocarbons such as toluene, xylene, ethylbenzene and the like; aliphatic hydrocarbons such as n-pentane, n-hexane and the like; alicyclic hydrocarbons such as cyclopentane, cyclohexane and the like; halogenated aromatic hydrocarbons such as monochlorobenzene and the like; ether compounds such as dioxane, tetrahydrofuran and the like.

The used amount of the organic solvent is an amount capable of dissolving an amine compound and a carboxylic acid (2C) , and varies depending on the temperature at which an amine compound and a carboxylic acid (2C) are mixed in the first step, and 1 to 20 parts by weight with respect to 1 part by weight of the carboxylic acid (2C).

As the organic solvent, further, an alcohol solvent can also be added. The alcohol solvent includes, for example, alcohol solvents having 1 to 4 carbon atoms such as methanol, ethanol, isopropyl alcohol and the like.

The used amount of the alcohol solvent is usually 0 to 4 parts by weight with respect to 1 part by weight of the carboxylic acid (2C).

The organic solvent is more preferably a mixed solvent composed of a hydrocarbon solvent such as aromatic hydrocarbons, aliphatic hydrocarbons, alicyclic hydrocarbons and the like and an alcohol solvent having 1 to 4 carbon atoms, or is a hydrocarbon solvent such as aromatic hydrocarbons, aliphatic hydrocarbons, alicyclic hydrocarbons and the like, further preferably a mixed solvent composed of an aromatic hydrocarbon and an alcohol solvent having 1 to 4 carbon atoms, or is an aromatic hydrocarbon.

The temperature at which a carboxylic acid (2C) and an amine compound are mixed in the first step is usually 10 to 100°C, preferably 20 to 100°C, more preferably 20 to 60°C, further preferably 20 to 60°C.

The first step is carried out by, for example, a method in which an amine compound is added to a mixture of a carboxylic acid (2C) and an organic solvent, and the carboxylic acid (2C) and the amine compound are mixed, a method in which a carboxylic acid (2C) is added to a mixture of an amine compound and an organic solvent, and the carboxylic acid (2C) and the amine compound are mixed, a method in which a mixture of an organic solvent and an amine compound is added to a carboxylic acid (2C) , and the carboxylic acid (2C) and the amine compound are mixed, or a method in which a mixture of an organic solvent and a carboxylic acid (2C) is added to an amine compound, and the carboxylic acid (2C) and the amine compound are mixed. Addition in these methods can also be gradually carried out by divisional addition, dropping and the like.

The second step is a step of depositing a salt of an amine compound with a carboxylic acid (2) (hereinafter, referred to as present salt in some cases) from the mixture obtained in the first step.

In the second step, for example, the mixture obtained in the first step is cooled to a temperature lower than the temperature at which a carboxylic acid (2C) and an amine compound are mixed in an organic solvent in the first step, and preferably, the mixture is cooled to a temperature in the range of -20 to 30°C.

To equalize the particle size of the present salt to be deposited, the mixture obtained in the first step may be heated up to a temperature over the temperature at which a carboxylic acid (2C) and an amine compound are mixed in an organic solvent in the first step, then, cooled. In the case of performing such an operation, its frequency is not limited and preferably once or twice.

In the second step, the mixture obtained in the first step may be cooled down to a temperature in the above-described range while stirring, to cause deposition of the present salt, or the mixture may be cooled down to a temperature in the above-described range, then, allowed to stand still, to cause deposition of the present salt, and preferably, the mixture is cooled down to a temperature in the above-described range while stirring, to cause deposition of the present salt.

In the second step, if necessary, the mixture obtained in the first step may be condensed, or the organic solvent used in the first step may be further added.

In the method of the present invention, the second step can also be effected while carrying out the first step. Such an embodiment includes, specifically, a method in which an amine compound is added to a mixture of a carboxylic acid (2C) and an organic solvent, and the present salt is sequentially deposited from the resultant mixture while adding, and a method in which a carboxylic acid (2C) is added to a mixture of an amine compound and an organic solvent, and the present salt is sequentially deposited from the resultant mixture while adding. Such an embodiment is carried out usually in the range of -20 to 30°C.

For depositing the present salt, a seed crystal may be added to the mixture obtained in the first step.

Stirring or still standing for causing deposition of the present salt may advantageously be carried out, for example, until the solid of the present salt does not increase in the above-described temperature range, and the time necessary for stirring or still standing is, specifically, usually 10 minutes to 24 hours, preferably 30 minutes to 20 hours.

The third step is a step of recovering the present salt deposited in the second step. Recovery of the present salt is carried out by, for example, solid-liquid separation means such as decantation, filtration and the like, preferably carried out by filtration.

The filtration method includes, for example, gravity filtration, vacuum filtration, pressure filtration, centrifugal filtration, compression filtration and a combination of these filtration methods, and the like.

By the third step, a salt of an amine compound with a carboxylic acid (2) (present salt) is taken out as a solid, and a carboxylic acid (1) contained in a liquid phase such as filtration liquid and the like is separated from a carboxylic acid (2).

The above-described solid may contain other materials than the present salt such as a salt of a carboxylic acid (1) with an amine compound, and the like, and the content of the present salt in the above-described solid is preferably 70 wt% or more, more preferably 90 wt% or more.

Thus, a solid containing the present salt, preferably a solid containing a salt of at least one amine selected from the group consisting of t-butylamine, n-butylamine, hexylamine, cyclohexylamine and morpholine with a carboxylic acid (2) is a solid having low content of a salt of a carboxylic acid (1) , resultantly, a carboxylic acid (2) can be obtained efficiently by using a solid containing the present salt as the present salt in the fourth step described later.

The constitutional molar ratio of an amine compound to a carboxylic acid (2) in the present salt is usually 1:3 to 3:1, preferably 1:0.9 to 0.9:1.

For the carboxylic acid (2C), an amine compound is removed from a liquid phase such as filtration liquid and the like obtained in recovering the present salt in the third step, to obtain a carboxylic acid (1) (this step is referred to as recycle step in some cases) , and the above-described isomerization step is carried on the carboxylic acid (1) (this step is referred to as second isomerization step in some cases), thus, a carboxylic acid (2C) containing a carboxylic acid (2) obtained by isomerizing the carboxylic acid (1) can also be obtained. The carboxylic acid (2C) may be subjected to the first step again.

Specifically, since a liquid phase such as filtration liquid and the like obtained in recovering the present salt in the third step contains a carboxylic acid (1) , for example, such a liquid phase may be mixed with an aqueous alkali solution and/or a water-soluble acid to remove an amine compound, thereby recovering a carboxylic compound (1), then, the second isomerization step may be performed on the recovered carboxylic acid (1) , and a mixture containing the resultant carboxylic acid (2) may be subjected to the first step as the carboxylic acid (2C), or may be mixed with a separately prepared compound (2) and subjected to the first step. Further, it is also possible that a carboxylic acid (1) other than the above-recovered carboxylic acid (1) is mixed and the second isomerization step is performed.

In the recycle step, it is preferable that a liquid phase such as filtration liquid and the like containing a carboxylic acid (1) is mixed with a water-soluble acid and the mixture is separated into an oil layer and an aqueous layer, the aqueous layer is extracted with an organic solvent which is phase-separable from water such as toluene, xylene and the like, the extracted organic solvent layer and the oily layer are mixed, and the resultant mixed liquid is subjected to the second isomerization step. Further, the resultant mixed liquid may be purified by adsorption on activated carbon having an average pore diameter of about 3 nm such as CARBORAFFIN (trade mark of Japan EnviroChemicals, Ltd. ) and the like, then, subjected to the second isomerization step.

The fourth step is a step of mixing the salt obtained in the third step (present salt) with an aqueous alkali solution and/or a water-soluble acid. A carboxylic acid (2) can be obtained by mixing the present salt with an aqueous alkali solution and/or a water-soluble acid.

The water-soluble acid in the present invention means an acid which can form a uniform phase with water, and includes inorganic acid such as mineral acid such as sulfuric acid, hydrochloric acid and the like; and organic acids such as carboxylic acids having 1 to 3 carbon atoms such as acetic acid and the like, sulfonic acids having 1 to 3 carbon atoms such as methanesulfonic acid and the like. The water-soluble acid preferably includes an aqueous solution containing usually 1 to 35 wt%, preferably 5 to 20 wt% of hydrochloric acid, an aqueous solution containing usually 1 to 98 wt%, preferably 5 to 50 wt% of sulfuric acid, and the like.

The aqueous alkali solution in the present invention means an aqueous solution prepared by dissolving an alkali such as an alkali metal hydroxide, an alkali metal carbonate and the like in water. The alkali metal hydroxide includes sodium hydroxide, potassium hydroxide and the like, and the alkali metal carbonate includes sodium carbonate and the like. The concentration of an alkali contained in the aqueous alkali solution is usually 1 to 80 wt%, preferably 1 to 23 wt%.

The temperature at which the present salt and a water-soluble acid are mixed in the fourth step is usually 5 to 80°C.

pH of an aqueous phase obtained by mixing with a water-soluble acid is preferably 5 or less, more preferably in the range of 2 to 4. When pH of the above-described aqueous phase is over 5, it is preferable that an acid aqueous solution is further added and the mixture is stirred, then, allowed to stand still.

The temperature at which the present salt and an aqueous alkali solution are mixed in the fourth step is usually 10 to 80°C.

The used amount of an alkali is usually 0.6 to 1.5 mol, preferably 0.8 to 1.2 mol with respect to 1 mol of an amine compound.

The fourth step is carried out, preferably, by mixing the salt obtained in the third step with an aqueous alkali solution, then, further mixing with a water-soluble acid, and specifically, by the following methods (i) to (iv) . The fourth step is carried out, further preferably, by the following method (i) .
(i) An aqueous alkali solution is mixed with the present salt, if necessary, an organic solvent is added, and the mixture is separated into a layer containing an amine compound and an aqueous layer containing an alkali salt of a carboxylic acid (2) , the resultant aqueous layer is condensed if necessary, then, the above-described aqueous layer is dropped into a mixture of an organic solvent and an aqueous solution of a water-soluble acid, a carboxylic acid is extracted into an organic layer, to obtain a solution containing the carboxylic acid (2) . In this method, if necessary, a part or all of the organic solvent may be distilled off, to obtain a carboxylic acid (2) as a solid.
(ii) An aqueous alkali solution is mixed with the present salt, if necessary, an organic solvent is added, and the mixture is separated into a layer containing an amine compound and an aqueous layer containing an alkali salt of a carboxylic acid (2), the resultant aqueous layer is condensed if necessary, then, the above-described aqueous layer is dropped into an aqueous solution of a water-soluble acid, to cause deposition of the carboxylic acid (2) in the aqueous solution.
(iii) The solution containing the carboxylic acid (2) obtained by the above-described method (i) is cooled down to a temperature in the range of about 0 to about 50°C, preferably in the range of 0 to 20°C, to obtain the carboxylic acid (2).
(iv) An aqueous alkali solution is mixed with the present salt, if necessary, an organic solvent is added, and the mixture is separated into a layer containing an amine compound and an aqueous layer containing an alkali salt of a carboxylic acid (2), the above-described aqueous layer is dropped into an aqueous solution of a water-soluble acid, to obtain an aqueous solution containing the carboxylic acid (2).

The carboxylic acid (2) obtained in the fourth step may be, if necessary, subjected to, for example, solid-liquid separation such as filtration and the like, further, the resultant solid may be washed with a solvent in which the carboxylic acid (2) is poorly soluble. The resultant solid may be subjected to a purification treatment such as crystallization purification, extraction purification, distillation purification, adsorption purification on activated carbon, silica, alumina and the like, chromatographic methods such as silica gel column chromatography and the like; etc.

In the case of use of an organic solvent in the fourth step, the organic solvent includes aromatic hydrocarbons such as toluene, xylene, ethylbenzene and the like; aliphatic hydrocarbons such as n-pentane, n-hexane and the like; alicyclic hydrocarbons such as cyclopentane, cyclohexane and the like; acetates such as ethyl acetate and the like; etc., preferably the organic solvents used in the first step.

The content of a carboxylic acid (1) contained in thus obtained carboxylic acid (2) is in general 30 wt% or less, preferably 10 wt% or less, with respect to the total amount of the carboxylic acid (1) and the carboxylic acid (2).

The carboxylic acid (2) can be further subjected to an esterification reaction. Specific examples thereof include (a) a method in which a carboxylic acid (2) and a hydroxyl compound are dehydrated and condensed in the presence of a catalyst or in the absence of a catalyst, (b) a method in which a carboxylic acid (2) is converted into an acid halide with a halogenating agent such as thionyl chloride, phosphorus pentachloride and the like, and reacted with a hydroxy compound in the presence of a base catalyst such as pyridine and the like, (c) a method in which a carboxylic acid (2) is once converted into an esterified compound of a lower hydroxy compound having 1 to 4 carbon atoms according to the methods (a) and (b), and subjected to transesterification with a desired hydroxy compound (different from the above-described lower hydroxy compound) in the presence of a lithium compound, and other methods.

The method (a) is preferable, and a method of dehydration and condensation using a zirconium compound as a catalyst is more preferable.

Here, the hydroxy compound includes, for example, compounds represented by formula (3):

R²·OH (3)

(wherein, R2 and R are different groups, and represent a chain hydrocarbon group having 1 to 10 carbon atoms or a cyclic hydrocarbon group having 3 to 10 carbon atoms, optionally having at least one substituent selected from the following Group A.

Group A: halogen atoms, acyl groups having 2 to 7 carbon atoms optionally having a substituent, alkoxy groups having 1 to 7 carbon atoms optionally having a substituent, alkylthio groups having 1 to 3 carbon atoms, phenyl groups optionally having a substituent. )
(hereinafter, referred to as compound (3) in some cases), and the like.

Group A includes halogen atoms such as fluorine, chlorine, bromine, iodine and the like, a furyl group, a phenoxyfuryl group, a benzylfuryl group, a difluoromethyl group, a propargylfuryl group, a methylisoxazolyl group, a trifluoromethylthiazolyl group, a trifluoromethoxythiazolyl group, a propynylpyrrolyl group, a propynyldioxoimidazolydinyl group, an oxo group, a propenyl group, a propynyl group, a dioxotetrahydroisoindolyl group, an oxothiazolyl group and the like.

Compounds (3) in which R² represents a chain hydrocarbon group having 1 to 10 carbon atoms optionally having a substituent include methyl alcohol, ethyl alcohol, n-propyl alcohol, isopropyl alcohol, n-butyl alcohol, sec-butyl alcohol, tert-butyl alcohol, n-pentyl alcohol, neopentyl alcohol, amyl alcohol, n-hexyl alcohol, n-octyl alcohol, n-decyl alcohol, 2-furylmethyl alcohol, 3-furylmethyl alcohol, (5-phenoxy-3-furyl)methyl alcohol, (5-benzyl-3-furyl)methan-1-ol, {5-(difluoromethyl)-3-furyl}methan-1-ol, 5-propargylfurfuryl alcohol, (5-methylisoxazol-3-yl)methan-1-ol, 1-{2-(trifluoromethyl)-1,3-thiazol-4-yl}prop-2-yn-1-ol, 1-{2-(trifluoromethoxy)-1,3-thiazol-4-yl}prop-2-yn-1-ol, 1-{1-prop-2-ynyl-5-(trifluoromethyl)pyrrol-3-yl)prop-2-yn-1 -ol, (1-prop-2-ynylpyrrol-3-yl)methan-1-ol, 3-(hydroxymethyl)-1-propynyl-imidazolidine-2,4-dione, 4-hydroxy-3-methyl-2-(2-propenyl)-2-cyclopenten-1-one, 4-hydroxy-3-methyl-2-(2-propynyl)-2-cyclopenten-1-one, 2-(hydroxymethyl)-4,5,6,7-tetrahydroisoindole-1,3-dione, {1-(2-propynyl)pyrrol-3-yl}methan-1-ol, 5-(hydroxymethyl)-4-methyl-(2-propynyl)-1,3-thiazolin-2-one, 4-methylhept-4-en-1-yn-3-ol, chloromethyl alcohol, dichloromethyl alcohol, trichloromethyl alcohol, bromomethyl alcohol, dibromomethyl alcohol, tribromomethyl alcohol, fluoromethyl alcohol, difluoromethyl alcohol, trifluoromethyl alcohol, fluoroethyl alcohol, difluoroethyl alcohol, trifluoroethyl alcohol, tetrafluoroethyl alcohol, pentafluoroethyl alcohol, 3,3-dibromo-2-propen-1-ol, perfluoropropyl alcohol, hexafluoroisopropyl alcohol, perfluorobutyl alcohol, perfluoropentyl alcohol, perfluorohexyl alcohol, perfluorooctyl alcohol, perfluorodecyl alcohol, {1-(2-propynyl)-5-(trifluoromethyl)-4-pyrazolyl}methan-1-ol, 1-{1-(2-propynyl)-5-(trifluoromethyl)pyrrol-3-yl}prop-2-yn-1-ol, 1-{2-(trifluoromethyl)-1,3-thiazol-4-yl}prop-2-yn-1-ol, 1-{2-(trifluoromethoxy)-1,3-thiazol-4-yl}prop-2-yn-1-ol, 4-fluorohept-4-en-1-yn-3-ol and the like.

Compounds (3) in which R² represents a cyclic hydrocarbon group having 3 to 10 carbon atoms optionally having a substituent include, for example, benzyl alcohol, 2-methyl-3-phenylbenzyl alcohol, 2,3,5,6-tetrafluorobenzyl alcohol, 2,3,5,6-tetrafluoro-4-methylbenzyl alcohol, 2,3,5,6-tetrafluoro-4-methoxybenzyl alcohol, 2,3,5,6-tetrafluoro-4-(methoxymethyl)benzyl alcohol, 2,3,5,6-tetrafluoro-4-allylbenzyl alcohol, 2,3,5,6-tetrafluoro-4-propargylbenzyl alcohol, 2,3,5,6-tetrafluoro-4-(methylthiomethyl)benzyl alcohol, 2,3,5,6-tetrafluoro-4-(difluoromethyl)benzyl alcohol, 2,3,5,6-tetrafluoro-4-(difluoromethoxy)benzyl alcohol, 2,3,5,6-tetrafluoro-4-(2,2,2-trifluoroacetyloxy)methylbenzy 1 alcohol, 4-(trifluoromethyl)benzyl alcohol, 2,3,4,5-tetrafluoro-6-methylbenzyl alcohol, 3-phenylbenzyl alcohol, 2, 6-dichlorobenzyl alcohol, 3-phenoxybenzyl alcohol, 2-hydroxy-2-(3-phenoxyphenyl)ethanenitrile, 2-hydroxy-2-{4-(methoxymethyl)phenyl}ethanenitrile, 2-{3-(4-chlorophenoxy)phenyl}-2-hydroxyethanenitrile, 2-(4-amino-2,3,5,6-tetrafluorophenyl)-2-hydroxyethanenitril e, 2-(4-fluoro-3-phenoxyphenyl)-2-hydroxyethanenitrile, (2-methylphenyl)methyl alcohol, (3-methylphenyl)methyl alcohol, (4-methylphenyl)methyl alcohol, (2,3-dimethylphenyl)methyl alcohol, (2,4-dimethylphenyl)methyl alcohol, (2,5-dimethylphenyl)methyl alcohol, (2,6-dimethylphenyl)methyl alcohol, (3,4-dimethylphenyl)methyl alcohol, (2,3,4-trimethylphenyl)methyl alcohol, (2,3,5-trimethylphenyl)methyl alcohol, (2,3,6-trimethylphenyl)methyl alcohol, (3,4,5-trimethylphenyl)methyl alcohol, (2,4,6-trimethylphenyl)methyl alcohol, (2,3,4,5-tetramethylphenyl)methyl alcohol, (2,3,4,6-tetramethylphenyl)methyl alcohol, (2,3,5,6-tetramethylphenyl)methyl alcohol, (pentamethylphenyl)methyl alcohol, (ethylphenyl)methyl alcohol, (propylphenyl)methyl alcohol, (isopropylphenyl)methyl alcohol, (butylphenyl)methyl alcohol, (sec-butylphenyl)methyl alcohol, (tert-butylphenyl)methyl alcohol, (pentylphenyl)methyl alcohol, (neopentylphenyl)methyl alcohol, (hexylphenyl)methyl alcohol, (octylphenyl)methyl alcohol, (decylphenyl)methyl alcohol, (dodecylphenyl)methyl alcohol, (tetradecylphenyl)methyl alcohol, naphthylmethyl alcohol, anthracenylmethyl alcohol, 1-phenylethyl alcohol, 1-(1-naphthyl)ethyl alcohol, 1-(2-naphthyl)ethyl alcohol, 4-prop-2-ynylphenyl)methan-1-ol, 3-prop-2-ynylphenyl)methan-1-ol, (1-prop-2-ynyl-2-methylindol-3-yl)methan-1-ol, {1-prop-2-ynyl-2-(trifluoromethyl)indol-3-yl}methan-1-ol, 4-prop-2-enylindan-1-ol, 4-phenylindan-2-ol, 4-(2-thienyl)indan-2-ol, (2,3,6-trifluoro-4-pyridyl)methan-1-ol; alkoxyaralkyl alcohols obtained by optionally changing a halogen atom in the above-described haloaralkyl alcohols to methoxy, ethoxy, propoxy, isopropoxy, butoxy, sec-butoxy, tert-butoxy and the like; and cyanoaralkyl alcohols, nitroaralkyl alcohols, phenol, 1-naphthol, 2-naphthol, 4-prop-2-ynylphenol, 3-prop-2-ynylphenol, 4-hydroxyacetophenone, 4-hydroxybenzaldehyde, and those obtained by substituting an aromatic ring in these compounds by an alkyl group, an alkoxy group, a halogen atom or the like; etc.

The hydroxyl compound includes, preferably, primary alcohols, benzyl alcohol and the like, more preferably, 2,3,5,6-tetrafluoro-4-(methoxymethyl)benzyl alcohol, 2,3,5,6-tetrafluoro-4-methylbenzyl alcohol, 2,3,5,6-tetrafluoro-4-allylbenzyl alcohol, 2,3,5,6-tetrafluoro-4-propargylbenzyl alcohol, 2,3,5,6-tetrafluoro-4-(methylthiomethyl)benzyl alcohol and the like.

The used amount of the hydroxyl compound is usually 1 mol or more with respect to 1 mol of a carboxylic acid (2), and if necessary, the hydroxyl compound may be used in large excess, or may also be used as a solvent. In general, after completion of the esterification reaction, the unreacted hydroxyl compound can also be recovered by an operation such as, for example, distillation, extraction, liquid-separation and the like.

The zirconium compound includes Lewis acidic zirconium compounds and the like, preferably includes compounds represented by formula (5):

Zr(0)m(X)n(Y)4-2m-n (5)

(wherein, X and Y represent each independently a halogen atom, an alkoxy group having 1 to 10 carbon atoms, an acyloxy group having 2 to 10 carbon atoms, an acetylacetonate group, an amino group or a cyclopentadienyl group, m represents 0 or 1 and n represents 0, 1 or 2.).

Specific examples of the zirconium compound include zirconium tetrafluoride, zirconium tetrachloride, zirconium tetrabromide, zirconium tetraiodide, zirconium acetate, zirconium acetylacetonate, zirconium ethoxide, zirconium n-propoxide, zirconium i-propoxide, zirconium n-butoxide, zirconium t-butoxide, zirconium oxychloride, tetrakis(dimethylamino)zirconium, tetrakis (diethylamino) zirconium, zirconocene dichloride, zirconocene dimethoxide, decamethylzirconocene dichloride and the like, preferably zirconium tetrachloride and zirconium n-propoxide.

As the zirconium compound, commercially marketed anhydrides or hydrates can be used as they are. Complexes with a compound showing a coordinative property such as tetrahydrofuran, tetramethylethylenediamine and the like can also be used.

The used amount of the zirconium compound is usually about 0.001 to about 200 mol, preferably about 0.01 to about 10 mol with respect to 1 mol of a carboxylic acid (2).

The esterification reaction of a carboxylic acid (2) and a hydroxy compound (3) in the presence of a zirconium compound is preferably carried out under an atmosphere of an inert gas such as nitrogen and the like. The esterification reaction can be carried out at any of normal pressure, positive pressure and reduce pressure, and preferably, carried out at normal pressure or under reduced pressure. It is preferable that the esterification reaction is carried out while continuously removing water as a by-product of the dehydration condensation reaction out of the reaction system by a method such as distillation and the like.

The temperature of the esterification reaction is usually about 20 to about 200°C.

By the esterification reaction, a reaction mixture containing a cyanoalkenylcyclopropanecarboxylic acid ester represented by formula (4): 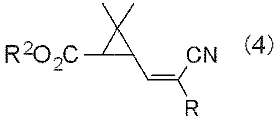 (wherein, R and R² represents the same meaning as described above.) (hereinafter, referred to as ester (4) in some cases.) can be obtained. The reaction mixture can be further washed with water or acidic water to remove a catalyst, and if necessary, may be subjected to a purification operation such as distillation, recrystallization, column chromatography and the like.

Thus obtained ester (4) can be used, for example, in pest control agents described in JP-A No. 2008-239597, and the like. Specific examples of the (Z)-cyanoalkenylcyclopropanecarboxylic acid ester include 4-methoxymethyl-2,3,5,6-tetrafluorobenzyl 3-(2-cyano-1-propenyl)-2,2-dimethylcyclopropanecarboxylate, 4-methyl-2,3,5,6-tetrafluorobenzyl 3-(2-cyano-1-propenyl)-2,2-dimethylcyclopropanecarboxylate, 4-allyl-2,3,5,6-tetrafluorobenzyl 3-(2-cyano-1-propenyl)-2,2-dimethylcyclopropanecarboxylate, 4-propargyl-2,3,5,6-tetrafluorobenzyl 3-(2-cyano-1-propenyl)-2,2-dimethylcyclopropanecarboxylate, 4-methylthiomethyl-2,3,5,6-tetrafluorobenzyl 3-(2-cyano-1-propenyl)-2,2-dimethylcyclopropanecarboxylate, 4-methoxymethyl-2,3,5,6-tetrafluorobenzyl 3-(2-chloro-2-cyanovinyl)-2,2-dimethylcyclopropanecarboxyla te, 4-methyl-2,3,5,6-tetrafluorobenzyl 3-(2-chloro-2-cyanovinyl)-2,2-dimethylcyclopropanecarboxyla te, 4-allyl-2,3,5,6-tetrafluorobenzyl 3-(2-chloro-2-cyanovinyl)-2,2-dimethylcyclopropanecarboxyla te, 4-propargyl-2,3,5,6-tetrafluorobenzyl 3-(2-chloro-2-cyanovinyl)-2,2-dimethylcyclopropanecarboxyla te, 4-methylthiomethyl-2,3,5,6-tetrafluorobenzyl 3-(2-chloro-2-cyanovinyl)-2,2-dimethylcyclopropanecarboxyla te and the like, and those obtained by substituting an ester portion of these compounds by the corresponding hydroxy compound, and the like, and those obtained by substituting a cyanocyclopropanecarboxylic acid portion of these compounds by the corresponding carboxylic acid, and the like.

According to the method of the present invention, a high content carboxylic acid (2) can be provided, even if a carboxylic acid (2C) contains a carboxylic acid (1).

### EXAMPLES

The present invention will be illustrated based on examples below. For the contents of a carboxylic acid (1) and a carboxylic acid (2) , a carboxylic acid (1) and a carboxylic acid (2) were separately prepared by crystallization and the like, and the contents were determined by a gas chromatography internal standard method, since different retention times are shown by respective gas chromatography procedures. <Production Example 1 of carboxylic acid (2C)>

Under a nitrogen atmosphere, to 129.7 of a xylene solution (concentration: 45.4 wt%) of methyl 2,2-dimethyl-3-[(1E)-2-formyl-1-propenyl]cyclopropanecarbox ylate were added 123. 2 g of a 24 wt% hydroxylamine hydrosulfate aqueous solution, 29.4 g of methanol and 11. 27 g of 80 wt% acetic acid, and 70.6 g of a 23 wt% sodium hydroxide aqueous solution was dropped at 21°C while keeping pH 5 over a period of 13 hours.

Thereafter, the reaction mixture was liquid-separated, and the resultant oil layer was washed with 58.76 g of water and 59. 12 g of water sequentially. To the resultant oil layer were added 6. 20 g of 35 wt% hydrochloric acid and 24 . 0 g of pyridine, and the mixture was stirred for 30 minutes, 75.9 g of acetic anhydride was dropped at 20°C over a period of 3 hours, then, the reaction solution was heated at 80°C and stirred for 14 hours. After cooling, to the reaction mixture were added 59 g of xylene and 276 g of water, and 14.8 g of 98 wt% sulfuric acid was dropped and the mixture was liquid-separated. The resultant aqueous layer was extracted with 58.27 g of xylene, combined with the oil layer obtained previously, and washed with 112 g of a 7 wt% sodium carbonate aqueous solution and 59 g of water sequentially. Thereafter, the oil layer was condensed under a reduced pressure of 3.0 kPa, to obtain 66.89 g of a solid containing methyl 2,2-dimethylcyclopropane-3-(2-cyano-1-propenyl)carboxylate. The content of 2,2-dimethyl-3-(2-cyano-1-propenyl)cyclopropanecarboxylic acid in the solid (meaning the total content of all steric isomers without differentiating steric isomers of the acid) was 76.1 wt% (gas chromatography internal standard method), and the yield based on methyl 2,2-dimethyl-3-[(1E)-2-formyl1-propenyl]cyclopropanecarboxy late was 88.0%.

Under a nitrogen atmosphere, to 65 g of the above-described solid was added 74 g of heptane, and the solid was dissolved at 57°C. The solution was cooled down to 38°C over a period of 45 minutes, a seed crystal was added at the same temperature, then, the mixture was cooled down to 33°C. The mixture was stirred for 1 hour at the same temperature, further cooled down to 1.3°C over a period of 3 hours. The mixture was stirred at the same temperature for 1 hour, then, filtrated under reduced pressure, and the wet cake was sequentially substituted and washed with cold heptane. After drying under reduced pressure, 50.8 g of a cake containing methyl 2,2-dimethyl-3-(2-cyano-1-propenyl)cyclopropanecarboxylate was obtained. The content of methyl (2,2-dimethyl-3-(2-cyano-1-propenyl)cyclopropanecarboxylate in the cake (meaning the total amount of all steric isomers without differentiating steric isomers of the methyl ester) was 92.1% (gas chromatography internal standard method), and the recovery ratio based on the above-described solid was 94.5%.

Under a nitrogen atmosphere, to 50.55 g of the above-described cake were added 44.24 g of methanol and 88.52 g of water, the mixture was heated up to 54°C and 41.85 g of a 23 wt% sodium hydroxide aqueous solution was dropped over a period of 1 hour, then, the mixture was stirred at the same temperature for 3 hours. Thereafter, 2.00 g of a 23 wt% sodium hydroxide aqueous solution was further added and the mixture was stirred for 1 hour. After cooling down to 20°C, 133 g of toluene and 115 g of water were added, and 13.33 g of 98 wt% sulfuric acid was dropped and the mixture was liquid-separated. To the resultant aqueous layer was added 88 g of toluene and extraction thereof was performed, combined with the oil layer obtained previously and washed with 45 g of water. Thereafter, the oil layer was partially condensed under a reduced pressure of 30 kPa, to obtain 130 g of a toluene solution containing 2,2-dimethyl-3-(2-cyano-1-propenyl)cyclopropanecarboxylic acid. The 2,2-dimethyl-3-(2-cyano-1-propenyl)cyclopropanecarboxylic acid was a mixture composed of a carboxylic acid represented by formula (1-1) and a carboxylic acid represented by formula (2-1) .

Hereinafter, the carboxylic acid represented by formula (1-1) is referred to as "carboxylic acid (1-1)" and the carboxylic acid represented by formula (2-1) is referred to as "carboxylic acid (2-1)" in some cases.

The total concentration of 2,2-dimethyl-3-(2-cyano-1-propenyl)cyclopropanecarboxylic acid (= total amount of all steric isomers) was 32.8 wt% (gas chromatography internal standard method), and the yield based on methyl (2,2-dimethyl-3-(2-cyano-1-propenyl)cyclopropanecarboxylate was 98.8%.

In the mixture, carboxylic acid (1-1) : carboxylic acid (2-1) = 98.9:1.1 [weight ratio], and the content of (1R,3R)-2,2-dimethyl-3-(2-cyano-1-propenyl)cyclopropanecarb oxylic acid with respect to 100 wt% of the sum of the carboxylic acid (1-1) and the carboxylic acid (2-1) was 97.5 wt%.

### Isomerization step 1

Under a nitrogen atmosphere, to 119 g of a toluene solution (concentration: 30 wt%) containing 2,2-dimethyl-3-(2-cyano-1-propenyl)cyclopropanecarboxylic acid obtained above was added 2 g of calcium chloride, then, 0.83 g of a toluene solution of 50 wt% t-butyl hydroperoxide and 2.85 g a 47 wt% hydrobromic acid aqueous solution were dropped simultaneously at 40°C over a period of 1 hour. After stirring at the same temperature for 3 hours, the reaction solution was cooled down to 25°C, distilled water was added to wash the solution and the mixture was liquid-separated. To the resultant oil layer was added 42 g of distilled water, and 38 g of a 23 wt% sodium hydroxide aqueous solution was dropped, then, the mixture was stirred for 30 minutes at an internal temperature of 45°C, and the mixture was separated into an oil layer I and an aqueous layer I. The oil layer I was further extracted with distilled water, to obtain an oil layer II and an aqueous layer II, and the aqueous layer I and the aqueous layer II were combined to obtain a sodium salt aqueous solution of 2,2-dimethyl-3-(2-cyano-1-propenyl)cyclopropanecarboxylic acid. To this sodium salt aqueous solution was added 54 g of toluene, concentrated sulfuric acid was dropped to cause liquid-separation to obtain an oil layer III and an aqueous layer III, toluene was added to the aqueous layer III and the toluene layer was extracted, and the oil layer III and the toluene layer were combined, to obtain an oil layer IV. The oil layer IV was washed with distilled water, then, dehydrated under reflux, to obtain 135 g of a toluene solution of 2,2-dimethyl-3-(2-cyano-1-propenyl)cyclopropanecarboxylic acid.

The total concentration of 2,2-dzmethyl-3-(2-cyano-1-propenyl)cyclopropanecarboxylic acid in the above-described toluene solution was 25.3 wt% (gas chromatography internal standard method).

The recovery ratio of a carboxylic acid (1-1) and a carboxylic acid (2-1) after the isomerization step 1 was 95.4% with respect to the amount before the isomerization step 1, and in the resultant toluene solution, carboxylic acid (1-1):carboxylic acid (2-1) = 39.2:60.8 [weight ratio].

Hereinafter, 2,2-dimethyl-3-(2-cyano-1-propenyl)cyclopropanecarboxylic acid obtained by the method described in the isomerization step 1 is referred to as coarse carboxylic acid 1 in some cases.

### (Example 1)

### <First step>

Under a nitrogen atmosphere, to a mixed solvent of 129 g of toluene and 26 g of methanol was added 8.14 g of t-butylamine, then, 130 g of a toluene solution of a coarse carboxylic acid 1 (carboxylic acid (1-1):carboxylic acid (2-1) = 39.2:60.8 [weight ratio], the total concentration: 25.3 wet%) was dropped at 20°C over a period of 3 hours, to obtain a mixture containing a t-butylamine salt of a carboxylic acid (1-1) and a t-butylamine salt of a carboxylic acid (2-1).

### <Second step>

The mixture obtained in the first step was stirred at 20°C for 1 hour, then, further stirred at 47°C for 1 hours and cooled down to 0°C over a period of 2 hours, and further stirred at 0°C for 1 hour, to obtain a mixture containing a t-butylamine salt (solid) of a carboxylic acid (2-1).

### <Third step>

The mixture obtained in the second step was filtrated under reduced pressure, and the resultant cake was washed with toluene cooled with ice. Thereafter, the mixture was dried in vacuo at 60°C, to obtain 22.7 g of a solid containing a t-butylamine salt of a carboxylic acid (2-1).

### <Fourth step>

Under a nitrogen atmosphere, 94 g of toluene, 45 g of distilled water and 22.12 g of the solid obtained in the third step were mixed, and 15 g of a 23 wt% sodium hydroxide aqueous solution was dropped gradually at 20°C. Subsequently, the mixture was stirred, then, liquid-separated to obtain an aqueous layer which was then condensed under a reduced pressure of 10 kPa until a vapor temperature of 42°C, thereby removing t-butylamine dissolved, then, 21 g of distilled water was added for dilution, to obtain a aqueous solution containing a sodium salt of a carboxylic acid (2-1).

Under a nitrogen atmosphere, 165 g of xylene, 4 g of distilled water and 11 g of concentrated sulfuric acid were mixed, and an aqueous solution containing the above-described sodium salt was dropped at 23°C over a period of 1 hour. After stirring for 30 minutes, to an organic layer obtained by liquid-separation was added 33 g of distilled water, and the mixture was further stirred, then, liquid-separated. The resultant organic layer was dehydrated under reflux under a reduced pressure of 10 kPa using a Dean·Stark drain tube, to obtain 192.27 g of a xylene solution containing a carboxylic acid (2-1). The xylene solution was condensed, to obtain 15.8 g of a mixture containing a carboxylic acid (2-1) as the main component.

The total concentration of a carboxylic acid (1-1) and a carboxylic acid (2-1) in the mixture was 94.8 wt% (gas chromatography internal standard method).

The total recovery ratio of 2,2-dimethyl-3-(2-cyano-1-propenyl)cyclopropanecarboxylic acid contained in the xylene solution obtained in the fourth step with respect to the total amount of 2,2-dimethyl-3-(2-cyano-1-propenyl)cyclopropanecarboxylic acid in the toluene solution of a coarse carboxylic acid 1 used in the first step was 49.9%.

The content rate of a carboxylic acid (1-1) to a carboxylic acid (2-1) in the xylene solution obtained in the fourth step:
carboxylic acid (1-1):carboxylic acid (2-1) = 3.2:96.8 [weight ratio]

### <Production Example 2 of carboxylic acid (2C)>

### Recycle step

Under a nitrogen atmosphere, to 288 g of the filtration liquid I obtained in the third step of the above-described Example 1 was added 38.7 g of water, then, 5.00 g of 98 wt% sulfuric acid was dropped at 21°C over a period of 1 hour, and further stirred at the same temperature for 30 minutes, then, liquid-separated into an oil layer I and an aqueous layer. To the resultant aqueous layer was added 60 g of toluene and the mixture was stirred for 30 minutes, and an oil layer ii was isolated. The oil layer i and the oil layer ii obtained by liquid-separation were mixed, to the resultant mixed liquid was added 14.96 g of water and the mixture was stirred for 30 minutes, and an oil layer iii was separated. To the oil layer iii were added 2.19 g of CARBORAFFIN (trade mark of Japan EnviroChemicals, Ltd.) as a kind of activated carbon and 2.25 g of water, and the mixture was dehydrated under total reflux under a reduced pressure of 30 kPa at 54 to 59°C. The solution obtained by dehydration was filtrated under reduced pressure, filter residues such as activated carbon and the like were washed with xylene, and combined to give a filtration liquid II. The filtration liquid II was condensed, to obtain 17.27 g of a mixture of a carboxylic acid (1-1) and a carboxylic acid (2-1).

The total concentration of the mixture (meaning the concentration of the total amount of all steric isomers) was 95.0 wt% (gas chromatography internal standard method). The yield was 46.7% based on the total amount of a carboxylic acid (1-1) and a carboxylic acid (2-1) contained in the filtration liquid I.

In the mixture, carboxylic acid (1-1):carboxylic acid (2-1) = 70.5:29.5 [weight ratio], and the content of (1R,3R)-2,2-dimethyl-3-(2-cyano-1-propenyl)cyclopropanecarb oxylic acid with respect to 100 wt% of the sum of a carboxylic acid (1-1) and a carboxylic acid (2-1) was 92.0 wt%.

### Isomerization step 2

Under a nitrogen atmosphere, to 15.57 g of the mixture obtained in the above-described recycle step and 21.94 g of a purified carboxylic acid (1) (purity: 99.3%, carboxylic acid (1-1):carboxylic acid (2-1) = 99.6:0.4 [weight ratio]) was added 82 g of toluene, then, 0.84 g of a toluene solution of 50 wt% t-butyl hydroperoxide and 6. 72 g of an acetic acid solution containing 20 wt% hydrogen bromide were dropped simultaneously at 40°C over a period of 1 hour. The mixture was stirred for 3.5 hours at the same temperature, then, the reaction solution was cooled down to 24°C, distilled water was added and the solution was washed and the mixture was liquid-separated. To the resultant oil layer was added 42 g of distilled water, and 47 g of a 23 wt% sodium hydroxide aqueous solution was dropped, then, the mixture was stirred at an internal temperature of 45°C for 30 minutes, to be separated into an oil layer I and an aqueous layer I.

The oil layer I was further extracted with distilled water, to obtain an oil layer II and an aqueous layer II, the aqueous layer I and the aqueous layer II were combined, to obtain a sodium salt aqueous solution of 2,2-dimethyl-3-(2-cyano-1-propenyl)cyclopropanecarboxylic acid. To this sodium salt aqueous solution was added 54 g of toluene, concentrated sulfuric acid was dropped to cause liquid-separation, thereby obtaining an oil layer III and an aqueous layer III, to the aqueous layer III was added toluene and the toluene layer was extracted, and the oil layer III and the toluene layer were combined, to obtain an oil layer IV. The oil layer IV was washed with distilled water, then, condensed, to obtain 35.23 g of a mixture containing a carboxylic acid (1-1) and a carboxylic acid (2-1) as the main component (hereinafter, referred to as coarse carboxylic acid 2 in some cases).

The total concentration of a carboxylic acid (1-1) and a carboxylic acid (2-1) in the above-described toluene solution was 99.9 wt% (gas chromatography internal standard method).

The recovery ratio after the isomerization step 2 was 98.2% based on a carboxylic acid (1-1) and a carboxylic acid (2-1) before the isomerization step 2, and in the coarse carboxylic acid 2, carboxylic acid (1-1): carboxylic acid (2-1) = 40.1:59.9 [weight ratio].

### (Example 2)

### <First step>

Under a nitrogen atmosphere, to a mixed solvent of 50.92 g of toluene and 11.86 g of methanol was added 4.58 g of t-butylamine, then, 60.96 g of a toluene solution containing the coarse carboxylic acid 1 was dropped at 23°C over a period of 3 hours, to obtain a mixture containing a t-butylamine salt of a carboxylic acid (1-1) and a t-butylamine salt of a carboxylic acid (2-1).

### <Second step>

The mixture obtained in the first step was stirred at 20°C for 1 hour, then, further stirred at 47°C for 1 hour, cooled down to 2°C over a period of 3 hours, and further stirred at 2°C for 1 hour, to obtain a mixture containing a t-butylamine salt (solid) of a carboxylic acid (2-1).

### <Third step>

The mixture obtained in the second step was filtrated under reduced pressure, and the cake was washed with toluene cooled with ice. Thereafter, the mixture was dried in vacuo at 60°C, to obtain 11.97 g of a solid containing a t-butylamine salt of a carboxylic acid (2-1). In the solid, [amine salt of carboxylic acid (1-1)]:[amine salt of carboxylic acid (2-1)] = 4.9:95.1 [weight ratio].
1H-NMR (CD3CD, TMS) δ(ppm): 1.19 (s, 3H), 1.29 (s, 3H), 1.35 (s, 9H), 1.69 (d, 1H), 1.92 (d, 3H), 2.26 (m, 1H), 6.03 (m, 1H)

### (Example 3)

### <First step>

A mixture of 10.0 g of a toluene solution containing a coarse carboxylic acid 1 (total concentration of carboxylic acid (1-1) and carboxylic acid (2-1) in toluene solution: 21.6 wt%, carboxylic acid (1-1): carboxylic acid (2-1)=39.2:60.8 [weight ratio]) and 7.9 g of toluene was placed under a nitrogen atmosphere, and further, 0.6 g of morpholine was dropped at 20°C.

### <Second step and Third step>

The mixture obtained in first step was stirred with heating up to 65°C, then, cooled down to 0°C.

The resultant mixture containing a morpholine salt of a carboxylic acid (1-1) and a morpholine salt of a carboxylic acid (2-1) was filtrated under reduced pressure, and the resultant cake was washed with toluene cooled with ice. Thereafter, the mixture was dried in vacuo at 60°C, to obtain 2.1 g of a solid containing a morpholine salt of a carboxylic acid (2-1) as the main component. A part of the solid was taken out, and the resultant salt was analyzed by gas chromatography to find that [morpholine salt of carboxylic acid (1-1)]:[ morpholine salt of carboxylic acid (2-1)] = 5.0:95.0 [weight ratio].

### (Example 4)

### <First step>

Into 42.58 g of a toluene solution containing a coarse carboxylic acid 1 (total concentration of carboxylic acid (1-1) and carboxylic acid (2-1) in toluene solution: 25.3 wt%, carboxylic acid (1-1): carboxylic acid (2-1)=38.9:61.1 [weight ratio]), 3.62 g of cyclohexylamine was dropped at 20°C over a period of 1 hour, and further, 30.30 g of toluene and 8.60 g of methanol were added.

### <Second step>

The mixture obtained in the first step was stirred at 47°C for 1 hour, then, cooled down to 2.5°C over a period of 3 hours, further, stirred at the same temperature for 16 hours, to obtain a mixture containing a cyclohexylamine salt of a carboxylic acid (2-1).

### <Third step>

The mixture obtained in the second step was filtrated under reduced pressure, to obtain 22.62 g of a solid containing a cyclohexylamine salt of a carboxylic acid (2-1).

### <Fourth step>

Under a nitrogen atmosphere, 24.5 g of toluene, 19 g of distilled water and 20.0 g of the solid obtained in the third step were mixed, and 6.23 g of a 23 wt% sodium hydroxide aqueous solution was dropped at 20°C over a period of 1 hour. Further, the mixture was stirred for 30 minutes, then, liquid-separated, to obtain an aqueous solution containing a sodium salt of a carboxylic acid (2-1).

Under a nitrogen atmosphere, 53.8 g of xylene and 3.68 g of 98% sulfuric acid were mixed, and the aqueous solution containing a sodium salt obtained above was dropped at 23°C over a period of 0.5 hours. Further, the mixture was stirred for 30 minutes, then, liquid-separated to obtain an organic layer to which 11 g of distilled water was added, the mixture was further stirred for 30 minutes, then, liquid-separated. The resultant organic layer was treated using an evaporator under a reduced pressure of 2.2 kPa to distill off cyclohexylamine and a solvent, obtaining 5.12 g of a mixture containing [carboxylic acid represented by formula (2-1)] as the main component.

The total concentration of a carboxylic acid (1-1) and a carboxylic acid (2-1) in the mixture was 98.6 wt% (gas chromatography internal standard method).

The total recovery ratio of a carboxylic acid (1-1) and a carboxylic acid (2-1) contained in the mixture obtained in the fourth step with respect to the total amount of a carboxylic acid (1-1) and a carboxylic acid (2-1) contained in the toluene solution used in the first step was 47.0%

The content rate of a carboxylic acid (1-1) to a carboxylic acid (2-1) in the mixture obtained in the fourth step:
carboxylic acid (1-1):carboxylic acid (2-1) = 11.7:88.3 [weight ratio]

Isomers with respect to a cyclopropane ring were analyzed by high performance liquid chromatography to obtain results as shown below, and the proportions of a cyclopropane trans form (1'), a cyclopropane cis form (1"), a cyclopropane trans form (2') and a cyclopropane cis form (2") of a carboxylic acid with respect to the total amount of a carboxylic acid (1) and a carboxylic acid (2) in the coarse carboxylic acid 1 (toluene solution used in Example 4), and the proportions of a cyclopropane trans form (1'), a cyclopropane cis form (1"), a cyclopropane trans form (2') and a cyclopropane cis form (2") of a carboxylic acid with respect to the total amount of a carboxylic acid (1) and a carboxylic acid (2) in the mixture obtained in Example 4, are shown below.

Isomer rate with respect to cyclopropane ring (before Example 4) (after Example 4)

| | |
|---|---|
| (1R,3R) form | 98.66% → 99.58% |
| (1R,3S) form | 0.54% → 0.25% |
| (1S,3R) form | 0.01% → 0.02% |
| (1S,3S) form | 0.78% → 0.15% |

### (Example 5)

### <First step>

Into 42.60 g of a toluene solution containing the coarse carboxylic acid 1 (total concentration of carboxylic acid (1-1) and carboxylic acid (2-1) in toluene solution: 25.3 wt%, carboxylic acid (1-1):carboxylic acid (2-1) = 38.9:61.1 [weight ratio]), 3.70 g of hexylamine was dropped at 20°C over a period of 1 hour.

### <Second step>

The mixture obtained in the first step was stirred at 47°C for 1 hour, then, cooled down to 2.5°C over a period of 3 hours, further, stirred at the same temperature for 16 hours, to obtain a mixed solution containing a hexylamine salt of a carboxylic acid (2-1).

### <Third step>

The mixture obtained in the second step was filtrated under reduced pressure, to obtain 7.87 g of a solid containing a hexylamine salt of a carboxylic acid (2-1).

### <Fourth step>

Under a nitrogen atmosphere, 24.5 g of toluene, 19 g of distilled water and 6.00 g of the solid obtained in the third step were mixed, and 3.04 g of a 23 wt% sodium hydroxide aqueous solution was dropped at 20°C over a period of 1 hour. Further, the mixture was stirred for 30 minutes, then, liquid-separated, to obtain an aqueous solution containing a sodium salt of a carboxylic acid (2-1).

Under a nitrogen atmosphere, 53.8 g of xylene and 3.61 g of 98% sulfuric acid were mixed, and the resultant aqueous solution was dropped at 19°C over a period of 2 hours. Further, the mixture was stirred for 30 minutes, then, liquid-separated to obtain an organic layer to which 11 g of distilled water was added, and the mixture was further stirred for 30 minutes, then, liquid-separated. The resultant organic layer was treated using an evaporator under a reduced pressure of 2.2 kPa to distill off hexylamine and a solvent, obtaining 2.61 g of a mixture containing a carboxylic acid (2-1) as the main component.

The total concentration of a carboxylic acid (1-1) and a carboxylic acid (2-1) in the mixture was 99.7 wt% (gas chromatography internal standard method).

The total recovery ratio of a carboxylic acid (1-1) and a carboxylic acid (2-1) contained in the mixture obtained in the fourth step with respect to the total amount of a carboxylic acid (1-1) and a carboxylic acid (2-1) contained in the toluene solution used in the first step was 24.2%.

The content rate of a carboxylic acid (1-1) to a carboxylic acid (2-1) in the mixture obtained in the fourth step:
carboxylic acid (1-1):carboxylic acid (2-1) = 2.0:98.0 [weight ratio]

The proportions of a cyclopropane trans form (1'), a cyclopropane cis form (1"), a cyclopropane trans form (2') and a cyclopropane cis form (2") of a carboxylic acid with respect to the total amount of a carboxylic acid (1) and a carboxylic acid (2) in the coarse carboxylic acid 1 (toluene solution used in Example 5), and the proportions of a cyclopropane trans form (1'), a cyclopropane cis form (1"), a cyclopropane trans form (2') and a cyclopropane cis form (2") of a carboxylic acid with respect to the total amount of a carboxylic acid (1) and a carboxylic acid (2) in the mixture obtained in Example 5, are shown below.

Isomer rate with respect to cyclopropane ring (before Example 5) (after Example 5)

| | |
|---|---|
| (1R,3R) form | 98.66% → 99.84% |
| (1R,3S) form | 0.54% → 0.13% |
| (1S,3R) form | 0.01% → 0.00% |
| (1S,3S) form | 0.78% → 0.02% |

### (Example 6)

### <First step>

Into 42.58 g of a toluene solution containing the coarse carboxylic acid 1 (total concentration of carboxylic acid (1-1) and carboxylic acid (2-1) in toluene solution: 25.3 wt%, carboxylic acid (1-1) : carboxylic acid (2-1)=38.1:61.1 [weight ratio]), 2. 68 g of n-butylamine was dropped at 22°C over a period of 1 hour.

### <Second step>

The mixture obtained in the first step was stirred at 47°C for 1 hour, then, cooled down to 2.5°C over a period of 3 hours, further, the mixture was stirred at the same temperature for 16 hours, to obtain a mixture containing a n-butylamine salt of a carboxylic acid (2-1).

### <Third step>

The mixture obtained in the second step was filtrated under reduced pressure, to obtain 2.27 g of a solid containing a n-butylamine salt of a carboxylic acid (2-1).

### <Fourth step>

Under a nitrogen atmosphere, 24.48 g of toluene, 18.75 g of distilled water and 1.25 g of the solid obtained in the third step were mixed, and 1. 06 g of a 23 wt% sodium hydroxide aqueous solution was dropped at 24°C over a period of 30 minutes. Further, the mixture was stirred for 30 minutes, then, liquid-separated, to obtain an aqueous solution containing a sodium salt of a carboxylic acid (2-1).

Under a nitrogen atmosphere, 53.79 g of xylene and 3.59 g of 98% sulfuric acid were mixed, and the resultant aqueous solution was dropped at 25°C over a period of 30 minutes. Further, the mixture was stirred for 30 minutes, then, liquid-separated to obtain an organic layer to which 10.83 g of distilled water was added, and the mixture was further stirred for 30 minutes, then, liquid-separated. The resultant organic layer was condensed, to obtain 0.72 g of a mixture containing a carboxylic acid (2-1).

The total concentration of a carboxylic acid (1-1) and a carboxylic acid (2-1) in the mixture was 92.0 wt% (gas chromatography internal standard method).

The total recovery ratio of a carboxylic acid (1-1) and a carboxylic acid (2-1) contained in the mixture obtained in the fourth step with respect to the total amount of a carboxylic acid (1-1) and a carboxylic acid (2-1) contained in the toluene solution used in the first step was 6.1%.

The content rate of a carboxylic acid (1-1) to a carboxylic acid (2-1) in the mixture obtained in the fourth step:
carboxylic acid (1-1):carboxylic acid (2-1) = 6.9:93.1 [weight ratio]

### (Reference Example 1)

A mixture of 25.7 g of 2,3,5,6-tetrafluoro-4-methoxymethylbenzyl alcohol, 0.2 g of BHT, 1.6 g of a 70 wt% zirconium tetrapropoxide propanol solution, 0.2 g of diisopropylethylamine and 135.2 g of xylene was distilled to remove propanol by-produced at a temperature of 144°C over a period of 7 hours. Next, 41.2 g of a xylene solution containing a carboxylic acid (1-1) and a carboxylic acid (2-1) obtained by the same manner as in Example 1 (carboxylic acid (1-1) : carboxylic acid (2-1) =3.3:96.7 [weight ratio], concentration: 50.9 wt%) and 5.7 g of xylene were added, and a Dean-Stark water separation tube was installed, and the mixture was stirred under reflux for 18 hours at 148°C while removing water by-produced during the reaction. Subsequently, 21.2 g of a 5 wt% sodium sulfate aqueous solution and 21.3 g of a 10 wt% sulfuric acid aqueous solution were added at 45°C and the mixture was washed, and liquid-separated. The resultant oil layer was subjected to the same washing procedure, then, 9.2 g of a 23 wt% sodium hydroxide aqueous solution and 33.3 g of water were added and the mixture was washed, and liquid-separated. The resultant oil layer was washed with 42.3 g of a 5 wt% sodium sulfate aqueous solution, and liquid-separated. The oil layer was condensed at 15 kPa, then, 77.2 g of water was added and the mixture was condensed again at 65 kPa. Thereafter, the pressure was reduced to 7 kPa to cause dehydration, obtaining 41.6 g of 4-methoxymethyl-2,3,5,6-tetrafluorobenzyl 3-(2-methyl-1-propenyl)-2,2-dimethylcyclopropanecarboxylate
Content: 97.0%
Yield: 92.2%.

### (Example 7)

### <First step>

Under a nitrogen atmosphere, to a mixed solvent of 266.9 g of toluene and 69.6 g of methanol was added 350. 0 g of a toluene solution containing the coarse carboxylic acid 1 (concentration: 24.5 wt%), then, 24.8 g of t-butylamine was dropped at 40°C over a period of 4 hours, to obtain a toluene solution containing a t-butylamine salt of a carboxylic acid (1-1) and a t-butylamine salt of a carboxylic acid (2-1).

### <Second step>

The toluene solution obtained in first step was heated up to 60°C over a period of 1 hour, and the mixture was stirred at the same temperature for 3 hours. Thereafter, the mixture was cooled down to 21°C over a period of 4 hours, and further stirred for 7 hours at 21°C, to obtain a mixed solution containing a t-butylamine salt (solid) of a carboxylic acid (2-1).

### <Third step>

The mixed solution obtained in the second step was filtrated under reduced pressure, and the cake was washed with toluene cooled with ice. Thereafter, the mixture was dried in vacuo at 50°C, to obtain 59.7 g of a solid containing a t-butylamine salt of a carboxylic acid (2-1). In the solid, [amine salt of carboxylic acid (1-1)]:[amine salt of carboxylic acid (2-1)] = 2.0:98.0 [weight ratio].

### (Example 8)

To a mixed solution of 732 mg of the coarse carboxylic acid 1 ([carboxylic acid represented by formula (1-14)] : [carboxylic acid represented by formula (2-14)] = 1:2 [weight ratio]), 5 ml of toluene and 1 ml of methanol was added t-butylamine at a temperature of 40°C at a proportion of 0.6 mol with respect to 1 mol of [carboxylic acid represented by formula (2-14)].

### <Second step>

The mixture obtained in the first step was cooled down to room temperature, to obtain a mixture containing a t-butylamine salt of [carboxylic acid represented by formula (2-14)].

### <Third step>

The mixture obtained in the second step was filtrated under reduced pressure, to obtain 432 mg of a solid containing a t-butylamine salt of [carboxylic acid represented by formula (2-14)]. [amine salt of carboxylic acid represented by formula (1-14)]:[amine salt of carboxylic acid represented by formula (2-14)] = 6:94 [weight ratio].

### Industrial Applicability

Carboxylic acids (2) such as (Z)-2,2-dimethyl-3-[2-cyano-1-propen-1-yl]cyclopropanecarbo xylic acid and the like are useful as a synthetic intermediate for agents of controlling pests such as harmful insects and the like. The method of the present invention can be industrially utilized as a method of a synthetic intermediate for agents of controlling pests such as harmful insects and the like.

## Claims

1. A method for producing a salt of an amine with a cyanoalkenylcyclopropanecarboxylic acid, the method comprising
a first step of mixing a carboxylic acid compound represented by formula (2C): 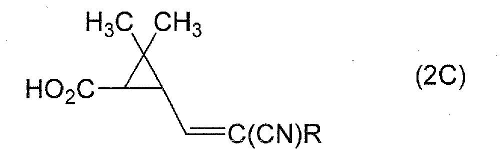 wherein, R represents an alkyl group optionally having a substituent, an alkylthio group or a halogen atom,
and at least one amine selected from the group consisting of primary amines and secondary amines in an organic solvent;
a second step of depositing a salt of the amine with a cyanoalkenylcyclopropanecarboxylic acid represented by formula (2): 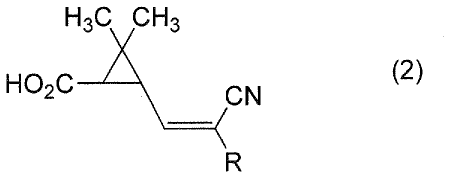 wherein, R represents the same meaning as described above, from the mixture obtained in the first step; and
a third step of recovering the salt deposited in the second step.

2. The method according to Claim 1, wherein the carboxylic acid compound represented by formula (2C) is a mixture of a cyanoalkenylcyclopropanecarboxylic acid represented by formula (2) and a cyanoalkenylcyclopropanecarboxylic acid represented by formula (1): 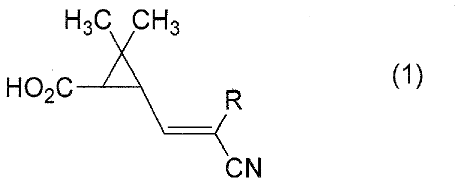 wherein, R represents the same meaning as described above.

3. The method according to Claim 1 or 2, wherein the amine is selected from the group consisting of t-butylamine, n-butylamine, hexylamine, cyclohexylamine and morpholine.

4. The method according to Claim 1 or 2, wherein the organic solvent is a mixed solvent composed of a hydrocarbon solvent and an alcohol solvent having 1 to 4 carbon atoms or a hydrocarbon solvent.

5. The method according to Claim 2, wherein the carboxylic acid compound represented by formula (2C) is one obtained via a step of isomerizing a cyanoalkenylcyclopropanecarboxylic acid represented by formula (1) into a cyanoalkenylcyclopropanecarboxylic acid represented by formula (2).

6. A salt of at least one amine selected from the group consisting of t-butylamine, n-butylamine, hexylamine, cyclohexylamine and morpholine with a cyanoalkenylcyclopropanecarboxylic acid represented by formula (2): 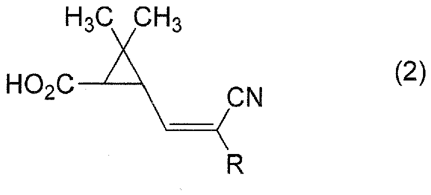 wherein, R represents an alkyl group optionally having a substituent, an alkylthio group or a halogen atom.

7. A method for producing a cyanoalkenylcyclopropanecarboxylic acid, the method comprising a step of mixing a salt of an amine with a cyanoalkenylcyclopropanecarboxylic acid represented by formula (2): 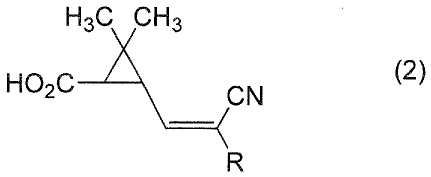 wherein, R represents an alkyl group optionally having a substituent, an alkylthio group or a halogen atom,
the salt having been obtained by the method according to Claim 1 or 2, and an aqueous alkali solution and/or a water-soluble acid.
